# EUROPEAN PATENT APPLICATION

(11) **EP 1 533 309 A1**
(43) Date of publication of application: **25.05.2005**
(21) Application number: 04025760.2
(22) Date of filing: 29.10.2004
(51) Int. Cl.: C07D 311/72

(54) **Method of producing a tocopherol product**

(30) Priority: 24.11.2003 US 720452
(71) Applicant: Deseret Laboratories, Inc., St George, UT 84790 (US)
(72) Inventor: Gubler, Scott, St George, Utah 84790 (US); Gonzalez, Angel, Ivins, Utah 84738 (US); Beatty, Rick, Hurricane, Utah 84737 (US)
(74) Representative: Adam, Holger, Dr.

(57) **Abstract**

A method for making a tocopherol product is provided. The method includes providing a desired amount of tocopheryl succinate substance and mixing a binder with the tocopheryl succinate substance to produce a mixture. A solution is sprayed on the mixture while contained in a granulator. The solution is then mixed with the mixture in the granulator. The mixture is dried so that a predetermined amount of the moisture content from the mixture is removed.

## Description

### BACKGROUND OF THE INVENTION

### 1. The Field of the Invention

The present invention generally relates to a method of producing a tocopheryl succinate product.

### 2. The Relevant Technology

Vitamin E supplements are popular as an adjunct to a modem diet. A common supplement containing Vitamin E is tocopheryl succinate, which is a Vitamin E compound derived from tocopherol and succinic anhydride.

Naturally occurring tocopherol homologues are generally isolated from natural products, for example, vegetable oil sources by various combinations of procedures such as esterification, saponification, extraction, distillation, ion exchange, adsorption chromatography, precipitation of sterols and crystallization. The tocopherol concentrate, when isolated, will vary depending on the particular separation technique used and the vegetable source.

Tocopheryl succinate has poor flow properties and is waxy and tacky at room temperature, making it difficult to handle in relatively pure form. In addition, the tocopheryl succinate products that are commercially available ordinarily have a broad particle size distribution with many fine particles. The uneven particle sizing causes the powder to be cohesive and to form clumps.

In U.S. Patent No. 6,130,343 to Hunsicker et al., which is incorporated herein by reference, a process of producing a coated tocopheryl succinate is disclosed. In the process, a tocopheryl succinate powder is mixed with a solution of a pharmaceutically acceptable binder. The tocopheryl succinate is maintained in a fluidized bed of a granulation apparatus during the mixing process by passing a fluidizing gas through the bed. Next, the solvent is evaporated from the tocopheryl succinate in the fluidized bed.

The temperature, however, of the fluidizing gas must remain sufficiently low when introduced into the bed. The process teaches that the temperature must be no higher than 30 degrees C such that the bed of tocopheryl succinate remains in a fluidized state during the mixing and evaporating steps. In addition, the particle size of the resultant product cannot be easily managed when using a fluidizing bed granulation apparatus to mix the tocopheryl succinate powder with the binder solution.

Various other methods have been used to produce Vitamin E products. In producing the products, however, methods are hampered by the oil based characteristics of tocopheryl succinate. Numerous attempts have been made to mix tocopheryl succinate with pharmaceutically acceptable binders, but the attempts have been unsuccessful. Usually when water-soluble binders are added to the tocopheryl succinate, the mixture produces large, unworkable clumps. Thus, the mixture is first dried before further processing the compound.

In view of the above and other related drawbacks and limitations identified in the relevant processes of tocopherol products, there is a need for a process that allows mixture of pharmaceutically acceptable binders with tocopheryl succinate while being able to manage the particle size of the resultant product.

### BRIEF SUMMARY OF THE INVENTION

In various exemplary embodiments of the present invention, a method for making a tocopherol product is provided. The method includes providing a desired amount of tocopheryl succinate substance and mixing a binder with the tocopheryl succinate substance to produce a mixture. A liquid is sprayed on the mixture while contained in a granulator. The liquid is then mixed with the mixture in the granulator to produce a resultant mixture. Next, the resultant mixture is dried by a predetermined amount.

These and other features of the present invention will become more fully apparent from the following description and appended claims, or may be learned by the practice of the invention as set forth hereinafter.

### BRIEF DESCRIPTION OF THE DRAWING

To further clarify the above and other advantages and features of the present invention, a more particular description of the invention will be rendered by reference to specific embodiments thereof which are illustrated in the appended drawings. It is appreciated that the drawings depict only typical embodiments of the invention and are therefore not to be considered limiting of its scope. The invention will be described and explained with additional specificity and detail through the use of the accompanying drawings in which:

Figure 1 is a schematic of an embodiment of a method for producing a tocopherol product in accordance with the present invention;

Figure 2 is a schematic of another embodiment of a method for producing a tocopherol product in accordance with the present invention; and

Figure 3 is a schematic of a further embodiment of a method for producing a tocopherol product in accordance with the present invention.

### DETAILED DESCRIPTION OF THE PREFERRED EMBODIMENTS

Reference will now be made in detail to the preferred embodiments of the present invention, examples of which are illustrated in the accompanying drawings. The various exemplary embodiments provide methods of producing a tocopherol product.

The process of the various embodiments, commences by obtaining tocopheryl succinate in the form of a powder material, which can be provided in tablet or capsule form. It is a feature of the process to feed the tocopheryl succinate into a system including a granulator, such as a high shear granulator. The granulator is used to mix the tocopheryl succinate with a binder. Various binders may be used, for example, cellulose based binders. In one method, a liquid is sprayed onto the tocopheryl succinate, followed by a mixing step. Suitable liquids can include, for example, water, solvent, a solution, an aqueous material or the like. A solution includes any combination of water, solvent or organic solvent based materials. The solution can also include a binder material. The process can include dry mixing the binder with the tocopheryl succinate, mixing the binder into a solution and sprayed onto the tocopheryl succinate or a combination of both. The binder coats at least a portion of the surface of the tocopheryl succinate particles during the mixing step.

In the embodiments of the present invention, methylcellulose or ethylcellulose may be used as the cellulose based binder. The presence of a cellulose material increases the ability to mix tocopheryl succinate with a water-based solution. By increasing the amount of the cellulose binder in the mixture, the biological concentrate of tocopheryl succinate is effectively reduced. This is generally considered undesirable. Thus, it is advantageous to use just enough of the cellulose binder to tackify the water thereby maintaining a high concentration of Vitamin E. It is preferred to use less than about 3 percent by weight of methylcellulose or ethylcellulose.

It is a feature of the present invention that the method allows lesser amounts of cellulose binder to be used in the formation of a fmal Vitamin E product. For example, the present invention allows the formation of wet and dry blends of a binder material at about 0.2 to about 3 percent by weight to the powered tocopheryl succinate. More specifically, the binder material can be provided having a consistency of about 0.5 to about 1.5 percent by weight to the powered tocopheryl succinate, or even about 0.5 to 1.0 percent by weight to the powered tocopheryl succinate.

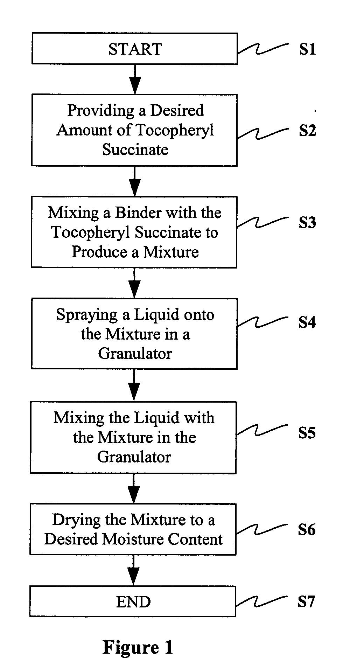

Figure 1 is a schematic representation of one process for making a tocopherol product in accordance with the present invention. The method starts in step S1 and continues to step S2 where a desired amount of a tocopherol-containing substance, shown as tocopheryl succinate, is provided. Step S3 involves mixing a binder, such as methylcellulose or ethylcellulose, with the tocopheryl succinate. Step S4 involves spraying a liquid on the mixture of tocopheryl succinate and binder, while still in the granulator. Next, in step S5, the liquid is mixed with the tocopherol/binder mixture in the granulator. Step S6 involves taking the resultant mixture of step S5 and drying it in order to reduce the moisture content by a predetermined amount. The moisture reduced can include, for example, moisture from water or a solvent. A predetermined amount can range between about 0.1 percent and about 1.4 percent, preferably between about 0.1 percent and about 0.5 percent. Finally, the process progresses to step S7 where the method ends.

As noted above, it is a feature of the present invention that the concentration of binder in step S3 may be between about 0.2 to about 1.5 percent by weight to the tocopheryl succinate. The spraying of the liquid in step S4 preferably applies a weight of solution of about 1 to about 40 percent of the total weight of the mixture.

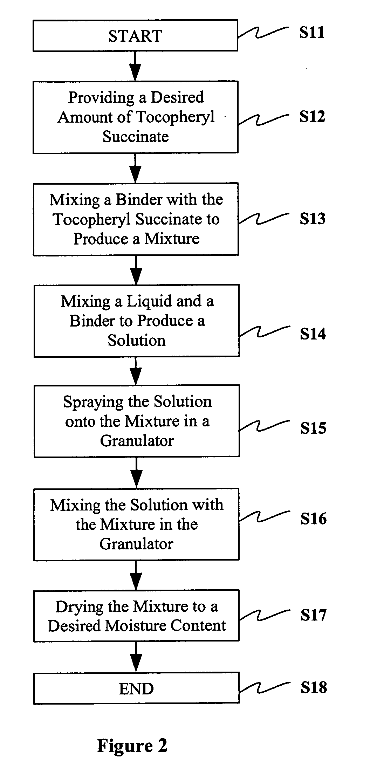

In Figure 2, another embodiment of the present invention is illustrated for making a tocopherol product. The method starts in step S11 and continues to step S12 where a desired amount of a tocopherol-containing substance, shown as tocopheryl succinate, is provided. The process then proceeds to step S 13 where a binder, such as methylcellulose, is mixed with the tocopheryl succinate. Step S14 involves mixing a liquid with a binder to produce a solution. Then, in step 15, the solution is sprayed on the mixture of tocopheryl succinate and binder, while still in the granulator. Next, step S16 involves mixing the solution with the tocopherol/binder mixture in the granulator. Step S 17 involves taking the resultant mixture of step S16 and drying it in order to reduce the moisture content by a predetermined amount. Finally, the process progresses to step S18 where the method ends.

In step S14 above, the additional binder may be selected from various known binders. It is presently preferred that the additional binder is the same type of binder as used in step S 13. The additional binder provides the ability to further tackify the water and reduce the water demand. The amount of additional binder may vary accordingly. For example, a solution of 0.1 percent by weight of methylcellulose may be added to the mixture.

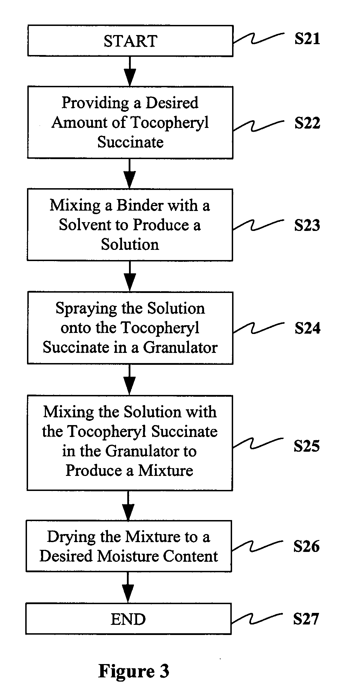

Figure 3 is a schematic representation of another process for making a tocopherol product in accordance with the present invention. The method starts in step S21 and continues to step S22 where a desired amount of a tocopherol-containing substance, shown as tocopheryl succinate, is provided. Step S23 involves mixing a binder, such as ethylcellulose, with a solvent, such as isopropyl alcohol, to produce a solution. In step S24, the solution is sprayed on the tocopheryl succinate while still in the granulator. Next, in step S25, the solution is mixed with the tocopherol/binder mixture from step S24 in the granulator. Step S26 involves taking the resultant mixture of step S25 and drying it in order to reduce the moisture content by a predetermined amount. Finally, the process progresses to step S27 where the method ends.

In the various embodiments of the invention, it is preferred that the solution be sprayed at a rate between about 50 to about 100 ml/s. Additionally, the method may further include heating water in the solution before mixing other components of the solution together to a temperature above about 80 degrees C or below about 90 degrees C, more specifically between about 80 to about 90 degrees C.

The method may also further include a step of establishing a bowl temperature in the granulator. For example, a bowl temperature in the granulator may be between about 15 to about 50 degrees C. More specifically, the bowl temperature may be between about 30 to about 32 degrees C.

The tocopheryl succinate used in the process of the various embodiments is initially in the form of a powder. The powdered tocopheryl succinate has a consistency of at least about 95 percent by weight, i.e., capable of passing through an 80 mesh sieve. The mesh size is defined by relevant U.S. standard as published in Handbook of Chemistry and Physics, p. F-158 (57th ed., CRC Press, Cleveland Ohio, 1976). The tocopheryl succinate typically consists essentially of particles having a relatively small average particle size, for example, an average particle size of less than about 1 millimeter.

The process spray-coats or dry blends the mixture containing the powdered tocopheryl succinate with the binder. When spray-coating is used in the process, the particle size is affected by the way in which the binder solution is sprayed. Particle size of the product is influenced by the spacing of the spray head from the bed, the rate of spray of binder into the bed and the amount of binder as a percentage of the product on a dry basis. The granulator promotes the formation of a larger particle sized product when the distance from the spray head to the bed is reduced, the rate of spray of binder into the bed is increased and the amount of binder in the product is increased. The granulator inhibits the formation of a larger particle sized product when the distance from the spray head to the bed is increased, the rate of spray of binder into the bed is reduced and the amount of binder in the product is reduced.

Water-soluble binders or organic solvent-soluble binders can be used as binders in the solution depending on the choice of solvent. There are various types of water-soluble binders, some of which include water-soluble celluloses, pregelatinized starches, and water-soluble macromolecules. Of those types, the water-soluble celluloses include hydroxypropylcellulose, hydroxymethylcellulose, hydroxypropylmethylcellulose, carboxymethylcellulose and methylcellulose. Reference to cellulose ethers is found in E. K. Just and T. G. Majewicz, "Cellulose Ethers", Encyclopedia of Polymer Science and Engineering, vol. 3, pp. 226-269 (John Wiley and Sons, Inc. N.Y., N.Y., 1985) the disclosure of which is incorporated herein by reference.

There are also various types of organic solvent-soluble binders, some of these include organic solvent-soluble cellulose derivatives, such as cellulose acetate phthalate, hydroxypropylmethylcellulose phthalate and ethylcellulose. Solvents can be used to dissolve the binders in the process. Examples of solvents include water and organic solvents, for example, alcohols such as methyl alcohol, ethyl alcohol, isopropyl alcohol, and ketones, such as acetone.

When ethylcellulose is used as a binder, the process involves spraying or dry mixing about 1 to about 2 percent by weight of ethylcellulose in a solvent mixture into a high shear granulator. Various levels of the ethylcellulose may be provided in the process. In addition, a solvent is later added to the mixture to dissolve the binder. The mixture is then dried in a fluid bed dryer after high shear granulation.

The granulation is carried out by spray-coating or dry mixing the tocopheryl succinate powder with a solution containing a binder. The powder is blended in the granulator until a precise particle size desired has been achieved. Typically, the solution of binder can be applied to the bed at a rate of about 0.01 to about 1.0 parts by weight of binder solution (on a basis of one hundred parts by weight of tocopherol succinate in the bed) per minute.

After granulation and evaporation of solvent, the granules of tocopheryl succinate and binder are removed from the granulator and placed in a stationary fluidizing bed to be dried. The mixture is dried by directing a continuous stream of fluidizing gas, such as air or nitrogen, through the bed. The product should be maintained in a fluidized state until the desired degree of evaporation has been obtained. The product should then be removed before attrition of the product occurs in the fluidized bed.

The inlet temperature of the fluidizing gas can be regulated. An elevated bed temperature can be monitored by measuring the product and outlet temperatures. The fluidizing gas used during the evaporation phase of the process should not be saturated in the solvent. Also, a dry gas used during the drying phase of the process can result in uneven drying in the bed. Thus, the process can introduce an air that is dehumidified into the bed and then rehumidify the air to a desired low humidity.

The tocopheryl succinate product can then be formed into tablets. Conventional methods of tabletting may be used. Typically, a lubricant and, if desired, another drug substance and/or an excipient, such as lactose, sucrose and mannitol can be added to the product. Examples of lubricants that may be used in the tablet manufacture include talc, stearic acid and stearates, such as magnesium stearate and calcium stearate. The lubricant is selected and added in a measure that provides practical strength and disintegration. The amount of lubricant typically is in the range of about 0.1 to about 7 percent by weight based on the main active substance. If a stearate or stearic acid is used, the typical amount can be at least about 0.5 percent by weight based on the main active substance. Tablet compression is typically about 1 to 2 ton/cm².

Examples discussed below illustrate various embodiments of the invention and should not be construed to limit the invention.

### EXAMPLES

### Example 1

In this example, a solution is mixed together using hot water and a methylcellulose binder. A granulator is charged with 0.250 kilograms (about 98.4 percent by weight) of tocopheryl succinate powder. A solution is mixed together containing 0.004 kilograms (about 1.6 percent by weight) of hydroxypropylmethylcellulose ("HPMC") (available from Dow Chemical Company, Midland, Mich., as Methocel E50), 0.032 kilograms (about 12.6 percent of total weight) of hot water and 0.064 kilograms (about 25.2 percent of total weight) of cold water.

The hot water is heated between about 80 to about 90 degrees C and mixed with the HPMC by mixing with a variable speed mixer at a slow speed until the clumps are broken down and/or the solution is smooth. The cold water is then added to the solution and mixed together until the solution turns clear. The rate of spray is between about 1 to 3 grams per minute.

The bed is fluidized after spraying is completed until a desired moisture of the product is achieved. The moisture of the product in the bed is tested periodically. When the moisture is about 0.2 percent by weight, the fluidization is stopped and the product is removed from the bed.

### Example 2

This example uses an ethylcellulose binder and a solvent solution. The granulator is charged with 0.250 kilograms (about 99 percent by weight) of tocopheryl succinate powder. A solution is mixed together containing 0.0025 kilograms (about 1 percent by weight) of ethylcellulose (available from Dow Chemical Company, Midland, Mich., as Ethocel 100) and 0.048 kilograms (about 19.0 percent of total weight) of isopropyl alcohol. First, the isopropyl alcohol is placed in a variable speed mixer. Next, the ethylcellulose is added and mixed together with the isopropyl alcohol until smooth.

The solution is allowed to sit overnight to dissolve the particles and to get a clear usable solution. After allowing the solution to sit, it is sprayed on the bed. The bed is fluidized after spraying is completed until a desired moisture of the product is achieved. The moisture of the product in the bed is tested periodically. When the desired moisture is achieved, the fluidization is stopped and the product is removed from the bed.

### Example 3

In this example, a solution including a methylcellulose is heated using a hot plate. The granulator is charged with 5.0 kilograms (about 98.8 percent by weight) of tocopheryl succinate powder. A solution is mixed together containing 0.06 kilograms (about 1.2 percent by weight) of HPMC (Methocel E50), 0.65 kilograms (about 12.8 percent of total weight) of hot water and 1.293 kilograms (about 25.6 percent of total weight) of cold water.

The hot water is placed in a container and heated between about 80 to about 90 degrees C using a hot plate. Next, the HPMC is added to the container and mixed with the hot water by mixing with a variable speed mixer at a slow speed until the clumps are broken down and/or the solution is smooth. The cold water is then added to the solution and mixed together until the solution turns clear. The solution is allowed to cool to 32 degrees C. The rate of spray is about 1.0 grams per minute. The bowl is sealed and pressurized to 900 mbar.

The impeller is positioned at a speed of about 50 rpm. The bowl temperature is 32 degrees C. After about 2 minutes and 45 seconds the material is discharged from the bowl and disposed into the stationary fluid bed bowl. The moisture of the product in the bed is tested periodically. When the moisture is about 1.4 percent by weight, the fluidization is stopped and the product is removed from the bed.

### Example 4

This example uses a solvent solution where an ethylcellulose binder is first placed in a bowl then the solvent is mixed in. A bowl of a high shear granulation machine (BOHLE VMA 20 unit) is charged with 5.0 kilograms (about 98.8 percent by weight) of tocopheryl succinate powder. A solution is mixed together containing 0.06 kilograms (about 1.2 percent by weight) of ethylcellulose (Ethocel 100) and 1.03 kilograms (about 20.4 percent of total weight) of isopropyl alcohol.

First, the ethylcellulose is placed in a vessel. Next, the isopropyl alcohol is mixed in using a medium agitator. The solution is covered and allowed to sit overnight. The spray nozzles are at about 0.5 mm. The high shear granulator is then positioned at a bowl temperature of about 20 degrees C. The impeller is positioned to about 50 rpm and the vacuum is positioned at 900 mbar. About 0.5 kilograms of solution is weighed out and sprayed in at 6 minutes with the pump. The impeller mixes the solution until the isopropyl alcohol is evaporated. The remaining solution is then sprayed on the material. The bowl temperature is reduced to about 15 degrees C. The mixture is mixed until the isopropyl alcohol is evaporated.

### Example 5

This example dry mixes a tocopheryl succinate powder with a binder. A bowl of a high shear granulation machine (BOHLE VMA 20 unit) is charged with 5.0 kilograms (about 98.9 percent by weight) of tocopheryl succinate powder. Next, 0.055 kilograms (about 1.09 percent by weight) of HPMC (Methocel E50) is added to the bowl and dry mixed with the tocopheryl succinate for 1 minute at 100 rpm.

The bowl temperature is positioned to 32 degrees C. Then, the impeller is positioned to a speed of 50 rpm and the vacuum is positioned to 60 mbar. The mixture is sprayed with 0.327 kilograms (about 6.5 percent of total weight) of cold water and periodically mixed using the impeller. After mixing the materials for about 44 minutes, the mixture is placed in a stationary fluidizing bed. The granulated material is then dried with an inlet temperature of about 32 degrees C and an outlet temperature of about 31.5 degrees C. The moisture of the product in the bed is tested periodically. When the moisture is about 0.36 percent by weight, the fluidization is stopped and the product is removed from the bed.

### Example 6

In this example, a solution is mixed together using cold water. The example starts with about 0.315 kilograms (about 6.23 percent of total weight) of cold water, which is placed in a container. Next, 0.0064 kilograms of HPMC (Methocel E50) is added to the bowl and mixed with the cold water until the clumps are broken down and the solution is smooth. Then, a bowl of a high shear granulation machine (BOHLE VMA 20 unit) is charged with 5.0 kilograms of tocopheryl succinate powder and 0.49 kilograms of HPMC (Methocel E50).

The bowl temperature is positioned to 32 degrees C and the vacuum is positioned at 800 mbar. The tocopheryl succinate powder HPMC and binder are dry mixed using an impeller that is positioned at 200 rpm and a chopper is positioned at 50 percent for 1 minute to break up any clumps. The impeller is then positioned at 50 rpm without the chopper.

The solution is then sprayed over the material at a rate of about 50 to 100 ml/s periodically mixing the material in the bed. After mixing the materials for about 30 minutes and spraying the solution over the material, the mixture is placed in a stationary fluidizing bed. The granulated material is then dried with an inlet temperature of about 31 degrees C and an outlet temperature of about 31 to 32 degrees C. The moisture of the product in the bed is tested periodically. When the moisture is about 0.24 percent by weight, the fluidization is stopped and the product is removed from the bed.

### Example 7

A set of examples is provided that varies the mixing steps from that of Example 6. In these examples, a solution is mixed together by mixing about 0.252 to about 0.504 kilograms of water with about 0.00514 to about 0.0103 kilograms of HPMC (Methocel E50) in a container. The solution is mixed together until the clumps are broken down and the solution is smooth.

Then, a bowl of a high shear granulation machine (BOHLE VMA 20 unit) is charged with 4.0 kilograms of tocopheryl succinate powder and about 0.0337 to about 0.389 kilograms of HPMC (Methocel E50). The bowl temperature is positioned to 32 degrees C. The tocopheryl succinate powder and HPMC are dry mixed using an impeller that is positioned at 200 rpm for 1 minute to break up any clumps.

The impeller is then positioned at 50 rpm. The solution is then sprayed over the material for about 1 minutes and 46 seconds to about 1 minute and 49 seconds periodically mixing the material in the bed. After mixing the materials for about 3 minutes and spraying the solution over the material, the vacuum is positioned to 40 mbar and the bed is jogged every minute for 15 seconds for a total of about 30 to about 35 minutes.

The mixture is then placed in a stationary fluidizing bed. The granulated material is then dried with an inlet temperature of about 31.5 to about 34.4 degrees C and an outlet temperature of about 25.6 to about 30 degrees C. The moisture of the product in the bed is tested periodically. When the moisture is about 0.18 to about 0.88 percent by weight, the fluidization is stopped and the product is removed from the bed.

### Example 8

Example 8 uses a different grade of methylcellulose. A solution in this example is mixed together by mixing about 0.45 to about 0.5 kilograms (about 12.36 percent of total weight) of water with about 0.0103 to about 0.0107 kilograms (about 0.28 to about 0.32 percent by weight) of HPMC (Methocel E5) in a container. The solution is mixed together until the clumps are broken down and the solution is smooth.

Then, a bowl of a high shear granulation machine (BOHLE VMA 20 unit) is charged with about 3.6 to about 4.0 kilograms (about 98.85 to about 99.5 percent by weight) of tocopheryl succinate powder and about 0.0078 to about 0.337 kilograms (about 0.22 to about 0.83 percent by weight) of HPMC (Methocel E5). The bowl temperature is positioned to 32 degrees C. The tocopheryl succinate powder and HPMC are dry mixed together using an impeller that is positioned at 200 rpm for 1 minute to break up any clumps.

The impeller is then positioned at 50 rpm and the solution is sprayed over the material for about 1 minute and 49 seconds periodically mixing the material in the bed. After mixing the materials for about 3 minutes and spraying the solution over the material, the vacuum is positioned to 40 mbar and the bed is jogged every minute for 15 seconds for a total of 30 minutes.

The mixture is then placed in a stationary fluidizing bed. The granulated material is then dried with an inlet temperature of about 31.5 degrees C and an outlet temperature of about 30 degrees C. The moisture of the product in the bed is tested periodically. When the moisture is about 0.11 to about 0.49 percent by weight, the fluidization is stopped and the product is removed from the bed.

### Example 9

In this example, another variation in the grade of methylcellulose is used. A solution in this example is mixed together by mixing about 0.45 kilograms (about 12.36 percent of total weight) of water with about 0.0103 kilograms (about 0.28 percent by weight) of HPMC (Methocel E15) in a container. The solution is mixed together until the clumps are broken down and the solution is smooth.

Then, a bowl of a high shear granulation machine (BOHLE VMA 20 unit) is charged with about 3.6 kilograms (about 98.9 percent by weight) of tocopheryl succinate powder and about 0.0298 kilograms (about 0.82 percent by weight) of HPMC (Methocel E15). The bowl temperature is positioned to 32 degrees C. The tocopheryl succinate powder and HPMC are dry mixed together using an impeller that is positioned at 200 rpm for 1 minute to break up any clumps.

The impeller is then positioned at 50 rpm and the solution is sprayed over the material for about 1 minute and 49 seconds periodically mixing the material in the bed. After mixing the materials for about 3 minutes and spraying the solution over the material, the vacuum is positioned to 40 mbar and the bed is jogged every minute for 15 seconds for a total of 30 minutes.

The mixture is then placed in a stationary fluidizing bed. The granulated material is then dried with an inlet temperature of about 31.5 degrees C and an outlet temperature of about 30 degrees C. The moisture of the product in the bed is tested periodically. When the moisture is about 0.16 percent by weight, the fluidization is stopped and the product is removed from the bed.

### Example 10

This example uses ethylcellulose in a similar process to that described above in Example 9. A solution is mixed together by mixing about 0.0181 kilograms of an ethylcellulose (Ethocel 100) with about 0.706 kilograms of a denatured alcohol in a container. The solution is mixed together until the ethylcellulose is completely dissolved.

Next, a bowl of a high shear granulation machine (BOHLE VMA 20 unit) is charged with about 4.0 kilograms of tocopheryl succinate powder (Vitamin E 1210 Succinate). The bowl temperature is positioned to 32 degrees C. The tocopheryl succinate powder is dry mixed using an impeller that is positioned at 200 rpm for 1 minute to break up any clumps.

The impeller is then positioned at 50 rpm and the solution is sprayed over the material for about 2 minute and 46 seconds periodically mixing the material in the bed. After mixing the materials for about 3 minutes and spraying the solution over the material, the vacuum is positioned to 40 mbar and the bed is jogged every minute for 15 seconds for a total of 30 minutes.

The mixture is then placed in a stationary fluidizing bed after an initial drying phase. The granulated material is then dried with an inlet temperature of about 28 degrees C and an outlet temperature of about 30 degrees C. The moisture of the product in the bed is tested periodically. When the moisture is about 0.42 percent by weight, the fluidization is stopped and the product is removed from the bed.

### Example 11

In this example, a different grade of ethylcellulose is used in a similar process as that in Example 10. A solution is mixed together by mixing about 0.0181 kilograms of an ethylcellulose (Ethocel 10) with about 0.5 kilograms of a denatured alcohol in a container. The solution is mixed together until the ethylcellulose is completely dissolved.

Next, a bowl of a high shear granulation machine (BOHLE VMA 20 unit) is charged with about 3.6 kilograms of tocopheryl succinate powder. The bowl temperature is positioned to 32 degrees C. The tocopheryl succinate powder is dry mixed using an impeller that is positioned at 200 rpm for 1 minute to break up any clumps.

The impeller is then positioned at 50 rpm and the solution is sprayed over the material for about 1 minute and 49 seconds periodically mixing the material in the bed. After mixing the materials for about 3 minutes and spraying the solution over the material, the vacuum is positioned to 40 mbar and the bed is jogged every minute for 15 seconds for a total of 15 minutes.

The mixture is then placed in a stationary fluidizing bed. The granulated material is then dried with an inlet temperature of about 28 degrees C and an outlet temperature of about 28 degrees C. The moisture of the product in the bed is tested periodically. When the moisture is about 0.42 percent by weight, the fluidization is stopped and the product is removed from the bed.

### Example 12

Another grade of ethylcellulose is used in this example from that of Example 11. A solution is mixed together by mixing about 0.0181 kilograms of an ethylcellulose (Ethocel 45) with about 0.5 kilograms of a denatured alcohol in a container. The solution is mixed together until the ethylcellulose is completely dissolved.

Next, a bowl of a high shear granulation machine (BOHLE VMA 20 unit) is charged with about 3.6 kilograms of tocopheryl succinate powder (Vitamin E 1210 Succinate). The bowl temperature is positioned to 32 degrees C. The tocopheryl succinate powder is dry mixed using an impeller that is positioned at 200 rpm for 1 minute to break up any clumps.

The impeller is then positioned at 50 rpm and the solution is sprayed over the material for about 1 minute and 49 seconds periodically mixing the material in the bed. After mixing the materials and spraying the solution over the material, the vacuum is positioned to 40 mbar and the bed is jogged every minute for 15 seconds for a total of 15 minutes.

Once the initial drying phase is complete, the mixture is placed in a stationary fluidizing bed. The granulated material is then dried with an inlet temperature of about 28 degrees C and an outlet temperature of about 28 degrees C. The moisture of the product in the bed is tested periodically. When the moisture is about 0.49 percent by weight, the fluidization is stopped and the product is removed from the bed.

The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.

## Claims

1. A method for making a tocopherol product, comprising:
providing a desired amount of tocopheryl succinate substance;
mixing a binder with the tocopheryl succinate substance to produce a mixture; spraying a liquid onto the mixture in a granulator;
mixing the liquid with the mixture in the granulator; and
drying the resultant mixture by a predetermined amount.

2. The method of claim 1, wherein the step of mixing the binder is accomplished by dry mixing.

3. The method of claim 1, wherein the step of mixing the binder is performed in the granulator.

4. The method of claim 1, wherein the step of mixing the liquid with the mixture is accomplished in a high shear granulator.

5. The method of claim 1, wherein the liquid used in the step of spraying the liquid comprises water and a binder material.

6. The method of claim 5, further comprising the step of heating the water to a temperature above about 80 degrees C before spraying the liquid onto the mixture.

7. The method of claim 5, further comprising the step of heating the water to a temperature between about 80 to about 90 degrees C before spraying the liquid onto the mixture.

8. The method of claim 5, further comprising the step of heating the water to a temperature below about 90 degrees C before spraying the liquid onto the mixture.

9. The method of claim 5, further comprising the step of mixing the binder material and the water until the binder material becomes substantially dissolved.

10. The method of claim 1, wherein the liquid used in the step of spraying the liquid includes an organic solvent and a binder material.

11. The method of claim 10, further comprising the step of mixing the organic solvent and the binder material until the binder material is substantially dissolved.

12. The method of claim 1, wherein the liquid used in the step of spraying the liquid comprises an organic solvent.

13. The method of claim 1, wherein the liquid used in the step of spraying the liquid comprises about 1 to about 40 percent of the total weight of the mixture.

14. The method of claim 1, wherein the binder in the step of mixing the binder comprises about 0.2 to about 1.5 percent by weight of the tocopheryl succinate substance.

15. The method of claim 1, further comprising the step of establishing a bowl temperature in the granulator between about 15 to about 50 degrees C.

16. The method of claim 15, wherein the bowl temperature is between about 30 to about 32 degrees C.

17. The method of claim 1, wherein step of the drying the resultant mixture is accomplished by placing the resultant mixture in a stationary fluidizing bed.

18. The method of claim 1, further comprising the step of determining a moisture content of the mixture.

19. The method of claim 1, further comprising the step of tabletting the mixture after the step of drying the resultant mixture.

20. The method of claim 1, wherein the step of spraying the solution is accomplished in a high shear granulator.

21. A method for making a tocopherol product, comprising:
providing a desired amount of tocopheryl succinate substance;
mixing a binder with a liquid to produce a solution;
spraying the solution onto the tocopheryl succinate substance in a granulator;
mixing the solution with the tocopheryl succinate substance in the granulator to produce a mixture; and
drying the mixture by a predetermined amount.

22. The method of claim 21, wherein the step of mixing the solution is accomplished in a high shear granulator.

23. The method of claim 21, further comprising the step of mixing a binder material with the tocopheryl succinate substance before the step of spraying the solution.

24. The method of claim 21, wherein the step of mixing the binder with the liquid is mixed until the binder is substantially dissolved.

25. The method of claim 21, wherein the liquid used in the step of mixing the binder with the liquid comprises an organic solvent.

26. The method of claim 21, wherein the liquid used in the step of mixing the binder with the liquid comprises water.

27. The method of claim 21, wherein the solution used in the step of spraying the solution comprises about 1 to about 40 percent of the total weight of the mixture.

28. The method of claim 21, wherein the binder used in the step of mixing the binder comprises about 0.2 to about 1.5 percent by weight of the tocopheryl succinate substance.

29. The method of claim 21, wherein the step of drying the mixture is accomplished by placing the mixture in a stationary fluidizing bed.

30. The method of claim 21, further comprising the step of determining a moisture content of the mixture.

31. The method of claim 21, further comprising the step of tabletting the mixture after the step of drying the mixture.

32. The method of claim 21, wherein the step of spraying the solution is accomplished in a high shear granulator.

33. A tocopherol composition, comprising:
a binder in a concentration of about 0.2 to about 3 percent by weight of the composition;
a tocopheryl succinate substance in a concentration of at least about 97 percent by weight of the composition.

34. The composition of claim 33, wherein the binder comprises about 0.5 percent to about 1.5 percent by weight of the composition.

35. The composition of claim 34, wherein the binder comprises about 0.5 percent to about 1.0 percent by weight of the composition.

36. The composition of claim 33, wherein the tocopheryl succinate substance comprises about 97 percent to about 99.8 percent by weight of the composition.

37. The composition of claim 36, wherein the tocopheryl succinate substance comprises about 99 percent to about 99.8 percent by weight of the composition.

38. The composition of claim 37, wherein the tocopheryl succinate substance comprises about 99 percent to about 99.5 percent by weight of the composition.

39. The composition of claim 33, wherein the binder is a methylcellulose binder.

40. The composition of claim 33, wherein the binder is an ethylcellulose binder.

41. The composition of claim 33, wherein the binder is a hydroxypropylmethylcellulose binder.
